# FASCICULE DE BREVET EUROPEEN

(11) **EP 3 253 262 B1**
(45) Date de publication et mention de la délivrance du brevet: **06.06.2018**
(21) Numéro de dépôt: 16702090.8
(22) Date de dépôt: 29.01.2016
(51) Int. Cl.: A47G 25/90, A61B 42/50

(54) **DISPOSITIF POUR ENFILER LES GANTS EN MATIÈRE ÉLASTIQUE À USAGE UNIQUE STÉRILES OU NON STÉRILES**
VORRICHTUNG ZUM ANZIEHEN STERILER ODER NICHT STERILER EINWEGHANDSCHUHE AUS ELASTISCHEM MATERIAL
DEVICE FOR PUTTING-ON DISPOSABLE STERILE OR NON-STERILE GLOVES MADE OF AN ELASTIC MATERIAL

(30) Priorité: 03.02.2015 FR 1500223
(43) Date de publication de la demande: 13.12.2017
(73) Titulaire: Bereczky, Alexandre, 54720 Lexy (FR)
(72) Inventeur: Bereczky, Alexandre, 54720 Lexy (FR)
(74) Mandataire: Lecomte & Partners
(86) Numéro de dépôt international: PCT/EP2016/051866
(87) Numéro de publication internationale: WO 2016/124485

(56) Documents cités:
- JP-A- H0 253 902
- US-A- 4 002 276
- US-A- 4 915 272
- US-A1- 2008 073 388

## Description

La présente invention concerne un porte-gant pour enfiler les gants en matière élastique à usage unique, stériles ou non stériles. L'invention a également a trait à un dispositif pour enfiler les gants en matière plastique notamment.

La pose de ces gants en matière élastique est traditionnellement effectuée manuellement par l'utilisateur. Cette façon d'opérer qui prend du temps, risque de contaminer les gants et de transmettre des infections.

Le document de brevet publié JP H0253902 divulgue un dispositif pour enfiler des gants en matière élastique. Le dispositif prévoit de retourner le bord arrière du gant sur un anneau de renforcement et de clipper un anneau de maintien sur l'anneau de renforcement. Cet assemblage forme une surface d'appui stable du bord arrière du gant sur le contour d'une ouverture d'un caisson au sein duquel un vide est réalisé en vue de donner au gant une forme expansée, facilitant sa mise en place sur une main. Le retournement du bord arrière sur l'anneau de renforcement et la mise en place de l'anneau de maintien peut s'avérer fastidieuse et contraignante. De plus une fois le gant disposé sur la main, les anneaux de renforcement et de maintien peuvent s'avérer gênant.

Les documents de brevet publiés JP H01195851, US 4915 272 et US 4002276 divulguent des dispositifs pour enfiler des gants.

Dans chacun de ces enseignements, la fixation du bord arrière du gant au dispositif destiné à faciliter son enfilement requiert des efforts particuliers.

L'invention a pour objectif de pallier au moins un des inconvénients de l'état de la technique, en particulier de l'état de la technique susmentionné. Plus particulièrement, l'invention a pour objectif de proposer une solution pour enfiler facilement des gants en matière élastique qui soit pratique et économique.

L'invention a pour objet un porte-gant selon la revendication 1 et subsidiairement selon l'une des revendications 2 à 5. L'invention a également pour objet un ensemble selon la revendication 6 et subsidiairement selon l'une des revendications 7 et 8. L'invention a également pour objet un dispositif selon la revendication 9 et subsidiairement selon l'une des revendications 10 et 11.

Le porte-gant est formé d'un cylindre rigide creux avec un épaulement cylindrique sur sa face externe. D'un côté le porte-gant est muni d'une languette de positionnement et d'une rainure circulaire incomplète qui maintient le gant par le bourrelet cylindrique qui se situe à son extrémité. Le gant est maintenu de l'autre côté du porte-gant par un dispositif qui oppose une faible résistance mécanique au déploiement du gant. Ce dispositif pourrait être, par exemple, un disque souple muni de plusieurs coupures radiales.

La languette peut former une poignée de préhension du porte-gant, notamment lorsqu'il est utilisé sans caisson à vide.

Le dispositif comporte un caisson transparent relié à une pompe à dépression par un tuyau flexible. Le caisson présente sur sa face avant deux orifices circulaires autour desquels se trouvent des joints d'étanchéité. Les orifices circulaires accueillent les porte-gants.

Une fois les porte-gants mis en place dans les orifices circulaires du caisson, l'utilisateur fait gonfler les gants en appuyant sur la pédale de la pompe à dépression. Il introduit ses mains dans les gants et relâche la pédale, un ressort remet la pédale de la pompe à dépression à sa position initiale. Les gants se resserrent sur les mains de l'utilisateur, ensuite il pousse ses mains gantées en avant dans le caisson, la force ainsi exercée fait ressortir les bourrelets cylindriques des gants des rainures circulaires incomplètes des porte-gants. Les gants sont libérés. L'utilisateur ressort ses mains gantées du caisson.

Les dessins annexés illustrent l'invention :
La figure 1 représente un porte-gant vu de face, conforme à l'invention,
La figure 2 représente le porte-gant de la figure 1 suivant la coupe verticale illustrée à la figure 1,
La figure 3 représente le porte-gant de la figure 1 vu du dessus,
La figure 4 représente le porte-gant des figures 1 à 3 avec le gant déployé dans un caisson,
La figure 5 représente le caisson de la figure 4,
La figure 6 représente une pompe à pied destinée à être raccordée au caisson de la figure 5.

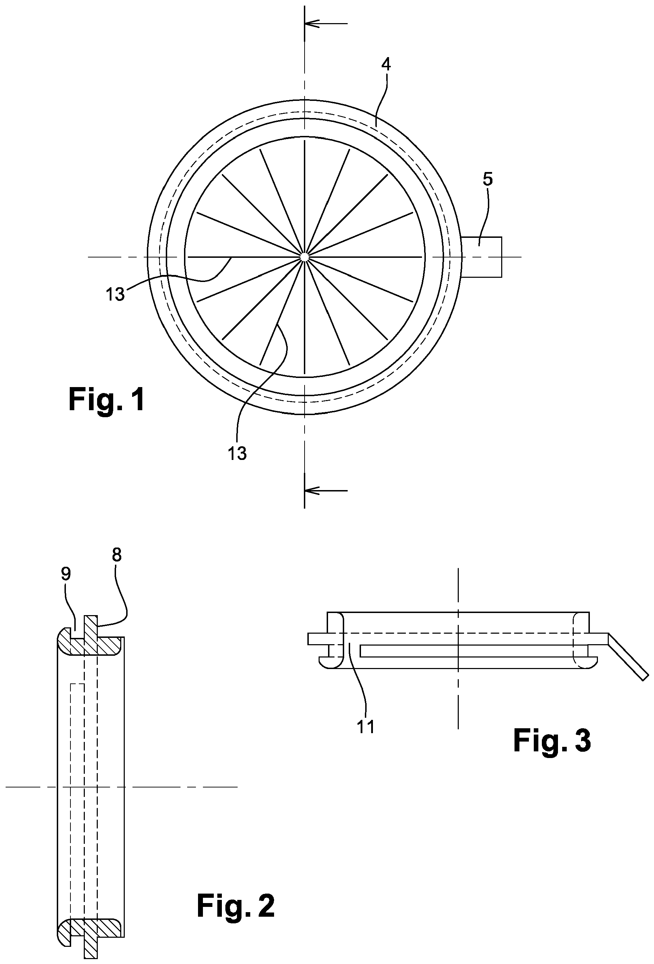

La figure 1 représente une porte-gant (4) conforme à l'invention, ledit porte-gant étant vu de face. Le porte-gant (4) est formé d'un cylindre rigide creux comprenant sur une face externe une languette de positionnement (5). Un disque souple muni de coupures radiales (13) peut être prévu pour maintenir le gant.

La figure 2 est une vue en coupe du porte-gant (4) de la figure 1. Le porte-gant comprend un épaulement cylindrique (8) sur sa face externe et une rainure circulaire incomplète (9) qui maintient le gant par son bourrelet cylindrique qui se trouve à son extrémité.

La figure 3 est une vue de dessus du porte-gant (4) de la figure 1. La partie non rainurée (11) facilite la désolidarisation du bourrelet cylindrique du gant par rapport au porte-gant (4) car à cet endroit, le bourrelet cylindrique du gant n'est pas maintenu par le porte-gant (4).

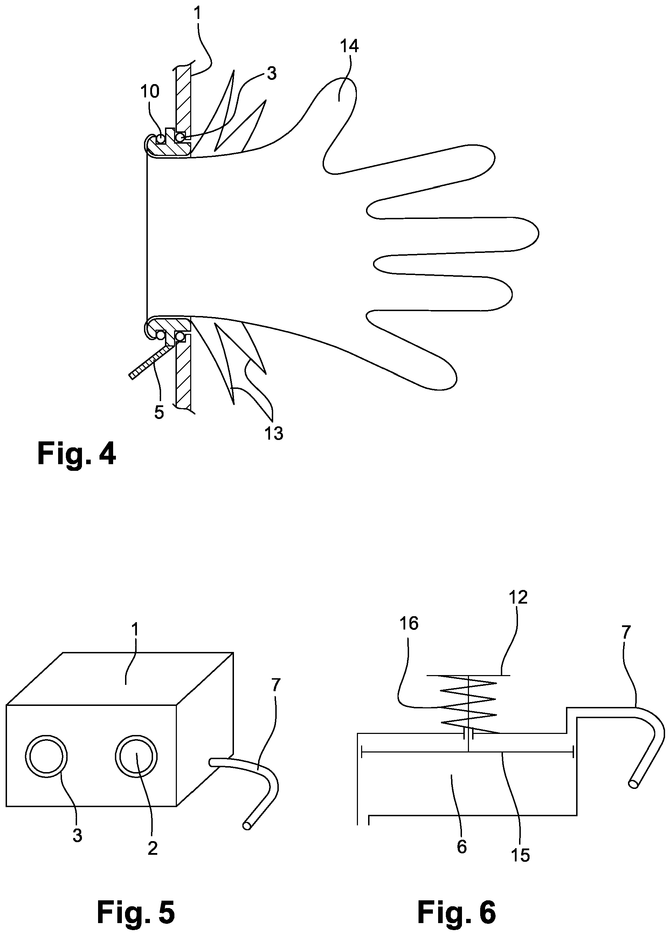

La figure 4 est une représentation du gant déployé. Le bourrelet cylindrique (10) est reçu dans la rainure du porte-gant. Le pouce (14) du gant à déployer est avantageusement positionné dans le haut du porte-gant (4) à l'opposé de la languette de positionnement (5). Le porte-gant (4) peut être maintenu manuellement par la languette (5). Lors du maintien manuel par la languette (5), le pouce de la main maintenant la languette (5) peut exercer une légère pression sur le bourrelet du gant lorsque l'autre main est insérée dans le gant, afin de stabiliser la retenue du bourrelet, et ensuite relâcher cette pression une fois que l'autre main est totalement insérée dans le gant. Le bourrelet du gant peut alors sortir de la rainure par l'effort appliqué par l'autre main dans la direction d'insertion dans le gant.

Alternativement le porte-gant (4) peut être monté de manière étanche, notamment au moyen des joints d'étanchéité (3), sur un caisson à vide (1) dont une partie de paroi est illustrée à la figure 4.

La figure 5 est une représentation du caisson (1) présentant sur sa face avant deux orifices circulaires (2) destinés à accueillir les porte-gants et autour desquels se trouvent des joints d'étanchéité (3). Le caisson est relié à une pompe à dépression par un tuyau flexible (7).

La figure 6 est une représentation de la pompe à dépression (6) qui est reliée au caisson par le tuyau (7). La pompe comprend une pédale (12) montée sur un ressort (16). La force exercée par l'utilisateur sur la pédale va faire descendre un piston (15) créant ainsi une dépression dans le caisson.

Les porte-gants (4) peuvent être introduits dans les orifices circulaires (2) à l'aide de leurs languettes de positionnement (5).

La force exercée par la différence de pression entre l'intérieur et l'extérieur du caisson (1) plaque les épaulements cylindriques (8) des porte-gants (4) contre les joints d'étanchéité (3) du caisson (1).

Cette force fait également céder les disques souples munis de plusieurs coupures radiales (13), qui maintenaient les gants. Les gants se déploient et gonflent, l'utilisateur y introduit ses mains et relâche la pédale (12) de la pompe à dépression (6). Le ressort (16) fait remonter le piston (15) de la pompe à dépression (6). Au fur et à mesure que la pression s'équilibre entre l'intérieur et l'extérieur du caisson (1) les gants se resserrent sur les mains de l'utilisateur. Ensuite il pousse en avant ses mains gantées dans le caisson (1). La force exercée au niveau de chaque gant permet à ce que la désolidarisation du bourrelet cylindrique (10) du gant par rapport au porte-gant (4) est facilitée par la partie non rainurée (11) du porte-gant (4) car à cet endroit, le bourrelet cylindrique (10) du gant n'est pas maintenu par le porte-gant (4).

Les gants sont libérés. L'utilisateur ressort ses mains gantées du caisson.

Lors du positionnement du porte-gant (4) dans l'orifice circulaire (2) du caisson (1) la languette de positionnement (5) est toujours située en bas de l'orifice circulaire (2).

Bien entendu, le mode de réalisation décrit ci-dessus, n'est pas limitatif. A titre d'exemples, le caisson (1) peut n'avoir qu'un orifice circulaire (2) pour ne recevoir qu'un porte-gant (4).

L'étanchéité entre le porte-gant (4) et le caisson (1) peut être faite par un assemblage conique ou sphéroconique.

Le dispositif selon l'invention, est particulièrement destiné aux endroits où il y a des risques de transmission d'infections en tous genres.

## Revendications

1. Porte-gant (4) pour enfiler un gant avec un bourrelet, notamment en matière élastique, comportant un cylindre rigide creux à travers lequel peut passer une main, **caractérisé en ce que** le cylindre rigide creux comporte sur une face externe une languette (5) de positionnement et une rainure (9) circulaire incomplète laissant une partie (11) non rainurée, destinée à recevoir le bourrelet (10) du gant, de sorte que lorsque l'utilisateur pousse sa main gantée vers l'avant, la force exercée fait ressortir le bourrelet de la rainure incomplète et libère le gant du porte-gant.

2. Porte-gant suivant la revendication 1, **caractérisé en ce qu'**il comporte du côté opposé au côté de la rainure (9) un dispositif destiné à maintenir le gant qui oppose une faible résistance mécanique au déploiement du gant.

3. Porte-gant suivant la revendication 2, **caractérisé en ce que** le dispositif qui oppose une faible résistance mécanique au déploiement du gant est constitué de plusieurs coupures (13) radiales.

4. Porte-gant suivant l'une des revendications 1 à 3, **caractérisé en ce qu'**il comporte un épaulement cylindrique (9) sur la face externe du cylindre rigide creux.

5. Porte-gant (4) suivant l'une des revendications 1 à 4, **caractérisé en ce que** la partie (11) non rainurée s'étend sur un secteur compris entre 5 et 30°, préférentiellement entre 10° et 20°.

6. Ensemble comportant un gant et un porte-gant suivant l'une des revendications précédentes, le gant comportant le bourrelet (10), notamment cylindrique, reçu dans la rainure (9) du porte-gant.

7. Ensemble suivant la revendication 6, **caractérisé en ce que** le bourrelet (10) est formé à l'extrémité du gant.

8. Ensemble suivant la revendication 6 ou 7, **caractérisé en ce que** le pouce du gant à déployer est positionné dans le haut du porte gant, à l'opposé de la languette (5) de positionnement.

9. Dispositif pour enfiler un gant en matière élastique comportant deux ensembles suivant l'une des revendications 6 à 8 et un caisson (1), notamment transparent, relié à une pompe à dépression (6), le caisson ayant sur une face avant deux orifices (2) accueillant chacun un ensemble suivant l'une des revendications 6 à 8.

10. Dispositif suivant la revendication 9, **caractérisé en ce que** la pompe comporte une pédale sur laquelle l'utilisateur exerce une force pour créer la dépression pour que les gants se déploient et gonflent.

11. Dispositif suivant la revendication 9 ou 10, **caractérisé en ce que** la force exercée par la différence de pression entre l'intérieur et l'extérieur du caisson plaque des épaulements (9) cylindriques des portes gants contre des joints d'étanchéité disposés autour des orifices circulaires.

## Patentansprüche

1. Handschuhhalter (4) zum Anziehen eines Handschuhs mit einem Wulst, der insbesondere aus elastischem Material hergestellt ist, umfassend einen hohlen starren Zylinder, durch den eine Hand hindurchpassieren kann, **dadurch gekennzeichnet, dass** der hohle starre Zylinder an einer Außenseite eine Positionierlasche (5) und eine unvollständige kreisförmige Nut (9), die einen nicht mit einer Nut versehenen Teil freilässt, die zur Aufnahme des Wulsts (10) des Handschuhs bestimmt ist, umfasst, sodass, wenn der Benutzer seine behandschuhte Hand nach vorne drückt, die ausgeübte Kraft den Wulst aus der unvollständigen Nut zwingt und den Handschuh von dem Handschuhhalter löst.

2. Handschuhhalter nach Anspruch 1, **dadurch gekennzeichnet, dass** er an der Seite, die der Seite der Nut (9) gegenüberliegt, eine zum Halten des Handschuhs bestimmte Vorrichtung umfasst, die dem Entfalten des Handschuhs einen leichten mechanischen Widerstand entgegensetzt.

3. Handschuhhalter nach Anspruch 2, **dadurch gekennzeichnet, dass** die Vorrichtung, die dem Entfalten des Handschuhs einen leichten mechanischen Widerstand entgegensetzt, aus einer Vielzahl radialer Schnitte (13) besteht.

4. Handschuhhalter nach einem der Ansprüche 1 bis 3, **dadurch gekennzeichnet, dass** er eine zylindrische Schulter (9) an einer Außenseite des hohlen starren Zylinders umfasst.

5. Handschuhhalter nach einem der Ansprüche 1 bis 4, **dadurch gekennzeichnet, dass** der nicht mit einer Nut versehene Teil (11) sich über einen Sektor von zwischen 5 und 30°, bevorzugt zwischen 10° und 20° erstreckt.

6. Anordnung, umfassend einen Handschuh und einen Handschuhhalter nach einem der vorhergehenden Ansprüche, wobei der Handschuh den Wulst (10), der insbesondere zylindrisch ist, in der Nut (9) des Handschuhhalters aufgenommen umfasst.

7. Anordnung nach Anspruch 6, **dadurch gekennzeichnet, dass** der Wulst (10) am Ende des Handschuhs ausgebildet ist.

8. Anordnung nach Anspruch 6 oder 7, **dadurch gekennzeichnet, dass** der Daumen des zu entfaltenden Handschuhs oben an dem Handschuhhalter angeordnet ist, gegenüber der Positionierlasche (5).

9. Vorrichtung zum Anziehen eines aus elastischem Material hergestellten Handschuhs, umfassend zwei Anordnungen nach einem der Ansprüche 6 bis 8 und einen Kasten (1), der insbesondere transparent ist, verbunden mit einer Vakuumpumpe (6), wobei der Kasten an einer Vorderseite zwei Öffnungen (2) aufweiset, die jede eine Anordnung nach einem der Ansprüche 6 bis 8 beherbergen.

10. Vorrichtung nach Anspruch 9, **dadurch gekennzeichnet, dass** die Pumpe ein Pedal umfasst, auf das der Benutzer eine Kraft ausübt, um das Vakuum zu erzeugen, sodass die Handschuhe sich entfalten und aufblähen.

11. Vorrichtung nach Anspruch 9 oder 10, **dadurch gekennzeichnet, dass** die durch den Druckunterschied zwischen dem Kasteninneren und dem Kastenäußeren ausgeübte Kraft zylindrische Schultern (9) der Handschuhhalter gegen um die kreisförmigen Öffnungen herum angeordnete Dichtungen presst.

## Claims

1. Glove holder (4) for putting on a glove having a bead, in particular made of elastic material, comprising a hollow rigid cylinder through which a hand can pass, **characterized in that** the hollow rigid cylinder comprises, on an outer face, a positioning tab (5) and an incomplete circular groove (9) leaving a non-grooved portion (11) that is designed to receive the bead (10) of the glove, such that when the user pushes their gloved hand forward the force exerted forces the bead out of the incomplete groove, and releases the glove from the glove holder.

2. Glove holder according to Claim 1, **characterized in that** it comprises, on the opposite side from the side of the groove (9), a device designed to hold the glove, which presents a slight mechanical resistance to the deployment of the glove.

3. Glove holder according to Claim 2, **characterized in that** the device which presents a slight mechanical resistance to the deployment of the glove consists of multiple radial cuts (13).

4. Glove holder according to one of Claims 1 to 3, **characterized in that** it comprises a cylindrical shoulder (9) on the outer face of the hollow rigid cylinder.

5. Glove holder according to one of Claims 1 to 4, **characterized in that** the non-grooved portion (11) extends over a sector of between 5 and 30°, preferably between 10° and 20°.

6. Assembly comprising a glove and a glove holder according to one of the preceding claims, the glove comprising the bead (10), which is in particular cylindrical, received in the groove (9) of the glove holder.

7. Assembly according to Claim 6, **characterized in that** the bead (10) is formed at the end of the glove.

8. Assembly according to Claim 6 or 7, **characterized in that** the thumb of the glove to be deployed is positioned at the top of the glove holder, opposite the positioning tab (5).

9. Device for putting on a glove made of elastic material, comprising two assemblies according to one of Claims 6 to 8 and a box (1), which is in particular transparent, connected to a vacuum pump (6), the box having on a front face two openings (2), each housing one assembly according to one of Claims 6 to 8.

10. Device according to Claim 9, **characterized in that** the pump comprises a pedal on which the user exerts a force so as to create the vacuum in order that the gloves deploy and inflate.

11. Device according to Claim 9 or 10, **characterized in that** the force exerted by the pressure difference between the box interior and the box exterior presses cylindrical shoulders (9) of the glove holders against seals arranged around the circular openings.
